# NEW EUROPEAN PATENT SPECIFICATION

(11) **EP 2 786 750 B2**
(45) Date of publication and mention of the opposition decision: **28.06.2023**
(45) Mention of the grant of the patent: 08.06.2016
(21) Application number: 12852587.0
(22) Date of filing: 30.11.2012
(51) Int. Cl.: A61K 31/198, A61K 31/44, A61K 45/00, A61P 35/00, A61P 43/00

(54) **AGENT FOR REDUCING ADVERSE SIDE EFFECTS OF KINASE INHIBITOR**
MITTEL ZUR VERRINGERUNG UNERWÜNSCHTER NEBENWIRKUNGEN VON KINASEHEMMERN
AGENT DESTINÉ À LA RÉDUCTION D'EFFETS SECONDAIRES NÉGATIFS D'UN INHIBITEUR DE KINASE

(30) Priority: 02.12.2011 JP 2011264687
(43) Date of publication of application: 08.10.2014
(73) Proprietor: EA Pharma Co., Ltd., Tokyo 104-0042 (JP); Yamaguchi University, Yamaguchi 753-8511 (JP)
(72) Inventor: YAMAMOTO, Naoki, Ube-shi Yamaguchi 755-8505 (JP); SAKAIDA, Isao, Ube-shi Yamaguchi 755-8505 (JP)
(74) Representative: Mewburn Ellis LLP
(86) International application number: PCT/JP2012/081213
(87) International publication number: WO 2013/081154

(56) References cited:
- WO-A1-2004/026294
- WO-A1-2004/058243
- JP-A- 2006 528 196
- HONG ET AL: "Curcumin ihnibits tyrosine kinase activity of p185neu ald also depletes p185neu", CLINICAL CANCER RES, vol. 5, 1 July 1999 (1999-07-01), - 1 July 1999 (1999-07-01), pages 1884-1891, XP002736358,
- AKIHARU WATANABE, SOSETSU: 'Kansaibo Gan no Eiyo Yobo' PHARMA MEDICA vol. 17, no. 3, 1999, pages 177 - 189, XP008173808
- ELEY,H.L. ET AL.: 'Effect of branched-chain amino acids on muscle atrophy in cancer cachexia' BIOCHEM. J. vol. 407, 2007, pages 113 - 120, XP008119488
- MASAFUMI IKEDA: 'Tokushu Gan Bunshi Hyoteki Chiryo no Genjo to Tenbo ''5. Rinsho de Bunshi Hyotekiyaku o Shiyo suru Sai no Point to Kadai 3) Kansaibo Gan''' PROG. MED. vol. 31, no. 11, 2011, pages 2607 - 2613, XP008173471
- MINORI ODANAKA ET AL.: 'Session 3-A Kansaibo Gan Kanja ni Okeru Sorafenib no Teashi Hifu Hanno no Risk Inshi' LIVER CANCER J. vol. 3, no. 2, 2011, pages 144 - 145, XP008173809
- SHIN'YA SUZUKI ET AL.: 'Note ''Evaluation of Improvement of Adherence Following Pharmacist Intervention for Hand/Foot Skin Reactions Induced by Sorafenib''' IRYO YAKUGAKU vol. 37, no. 5, 2011, pages 317 - 321, XP008173462
- COLLELA et al.: "Efficacy of a spray compound containing a pool of collagen precursor synthetic amino acids (L-proline, L-leucine, L-lysine and glycine) combined with sodium hyaluronate to manage chemo/radiotherapy-induced oral mucositis: preliminary data of an open clinical triaf?", Int. J. Immunopathology and pharmacology, vol. 23, no. 1, 2010, pages 143-151,

## Description

### TECHNICAL FIELD

The present invention relates to an agent for reducing the side effects of a kinase inhibitor, an anticancer medicine which combines branched-chain amino acids and a kinase inhibitor, and a medical kit or the like containing the same. Priority is claimed on Japanese Patent Application No. 2011-264687, filed December 2, 2011.

### BACKGROUND ART

Inhibitors of the various kinases, which participate in the proliferation of tumor cells and angiogenesis and the like, hold much promise as anticancer drugs. For example, the kinase inhibitor sorafenib, which exhibits in vivo inhibitory activity against a wide variety of kinases, is already in use clinically as an anticancer drug, particularly against hepatocellular carcinoma and renal cell carcinoma.

On the other hand, many anticancer drugs have significant side effects, and depending on the symptoms or severity of those side effects, administration of the anticancer drug may have to be abandoned in some cases. For example, ingestion of sorafenib causes hand-foot syndrome or bleeding from various tissues with relatively high frequency. Hand-foot syndrome is the generic name given to a side effect that occurs in the skin and nails of the hands and feet upon ingestion of the anticancer drug. The initial symptoms include sensory abnormalities such as numbness or tingling in the hands and feet, or the type of pain associated with burning, and in severe cases, causes intense pain that makes everyday life impossible. In this manner, the quality of life (QOL) of the patient is severely impaired, and therefore when hand-foot syndrome occurs, the administered dose of sorafenib must be reduced, or the administration of sorafenib must be temporarily or permanently halted. As a result, a method that reduces side effects while enabling the anticancer effect to manifest satisfactorily is keenly sought.

In some cases, using a combination of a plurality of anticancer drugs having different modes of action can yield a similar anticancer effect to that obtained by administering the agents individually, but with lower doses of the drugs. For example, Patent Document 1 discloses the use, as an anticancer drug, of a combination of a VEGFR tyrosine kinase inhibitor or a pharmaceutically acceptable salt thereof and a mTOR-selective kinase inhibitor or a pharmaceutically acceptable salt thereof, and lists sorafenib as one example of the VEGFR tyrosine kinase inhibitor.

On the other hand, it is known that branched-chain amino acids (BCAA) are effective against liver disease such as liver cancer. For example, Non-Patent Document 1 discloses that in a mouse model for non-alcoholic steatohepatitis (NASH), BCAA not only improved insulin resistance, but was also able to inhibit fatty change in the liver, and that administration of BCAA is therefore useful in preventing non-alcoholic fatty liver disease and inhibiting progression to NASH. Further, Patent Document 2 discloses that administration of BCAA can inhibit the development and progression of liver cancer. Moreover, the document also discloses that joint administration of BCAA and interferon enhances the anti-hepatitis C viral activity of interferon, and can also reduce the side effects of interferon such as fever.

In addition, Patent Document 4 discloses that a composition containing at least two or more types of essential amino acids, in which leucine in free or salt form is present in an amount of at least approximately 10 to 35% by weight based on the combined weight of all amino acids, is effective in improving cachexia (a state of severe malnutrition and negative nitrogen balance characterized by anorexia, namely loss of appetite, or severe weight loss and muscle atrophy) or weight loss caused by cancer.

### DOCUMENTS OF RELATED ART

### PATENT DOCUMENTS

Patent Document 1: Published Japanese Translation No. 2011-512395 of PCT
Patent Document 2: International Patent Publication No. 2004/058243
Patent Document 3: International Patent Publication No. 2007/013677
Patent Document 4: Published Japanese Translation No. 2006-503105 of PCT

### NON-PATENT DOCUMENTS

Non-Patent Document 1: Nemoto et al., "Investigation of the Utility of Branched-Chain Amino Acids (BCAA) in a Mouse Model for Non-Alcoholic Steatohepatitis (NASH)", The Japanese Journal of Gastroenterology, Vol. 105, Extra Edition, A832 (2008).

### SUMMARY OF THE INVENTION

### PROBLEMS TO BE SOLVED BY THE INVENTION

The aforementioned Patent Documents 1 to 4 and the Non-Patent Document 1 make absolutely no mention of a method that is capable of directly reducing the side effects of kinase inhibitors such as sorafenib.

The present invention has an object of providing an agent for reducing the side effects of kinase inhibitors such as sorafenib.

### MEANS TO SOLVE THE PROBLEMS

As a result of intensive investigation aimed at achieving the above object, the inventors of the present invention discovered that by jointly taking the kinase inhibitor sorafenib, which acts as an anticancer drug, and the branched-chain amino acids isoleucine, leucine and valine or a salt thereof, the side effects of sorafenib could be reduced, and they were therefore able to complete the present invention.

In other words, the present invention provides side effect-reducing agents (1) to (7), and anticancer medicines (8) to (14), all of which are described below.
(1) An agent for reducing a side effect of a kinase inhibitor, the agent containing all three branched-chain amino acids: isoleucine, leucine and valine or a salt thereof as an active ingredient, wherein the side effect is al least one of hand-foot syndrome and bleeding.
(2) The agent for reducing a side effect according to (1), wherein the kinase inhibitor is a kinase inhibitor of at least one kinase selected from the group consisting of KIT, FLT-3, RET, VEGFR-1, VEGFR-2, VEGFR-3, PDGFR-β and Raf.
(3) The agent for reducing a side effect according to (1) above, wherein the kinase inhibitor is 4-[4-[[4-chloro-3-(trifluoromethyl)phenyl]carbamoylamino]phenoxy]-N-methylpyridine-2-carboxamide, 4-[4-({[4-chloro-3-(trifluoromethyl)phenyl]carbamoyl}amino)-3-fluorophenoxy]-N-methylpyridine-2-carboxamide, N-(2-chloro-4-((6,7-dimethoxy-4-quinazolinyl)oxy)phenyl)-N'-propylurea, N-[3-[5-(2-aminopyrimidin-4-yl)-2-tert-butyl-1,3-thiazol-4-yl]-2-fluorophenyl]-2,6-difluorobenzenesulfonamide, 1-[1-[(2-aminopyridin-4-yl)methyl]indol-4-yl]-3-(5-bromo-2-methoxyphenyl)urea hydrochloride, N-[4-(3-amino-1H-indazol-4-yl)phenyl]-N'-(2-fluoro-5-methylphenyl)urea, 1-[2-chloro-4-(6,7-dimethoxyquinolin-4-yl)oxyphenyl]-3-(5-methyl-1,2-oxazol-3-yl)urea, 1-N'-[3-fluoro-4-[6-methoxy-7-(3-morpholin-4-ylpropoxy)quinolin-4-yl]oxyphenyl]-1-N-(4-fluorophenyl)cyclopropane-1,1-dicarboxamide, N-[[3-fluoro-4-[2-(1-methylimidazol-4-yl)thieno[3,2-b]pyridin-7-yl]oxyphenyl]carbamothioyl]-2-phenylacetamide, 1-N'-[2-fluoro-4-[2-[[4-(4-methylpiperazin-1-yl)piperidine-1-carbonyl]amino]pyridin-4-yl]oxyphenyl]-1-N-(4-fluorophenyl)cyclopropane-1,1-dicarboxamide, 4-[3-chloro-4-[[(cyclopropylamino)carbonyl]amino]phenoxy]-7-methoxy-monomethanesulfonate, 1-N'-(4-fluorophenyl)-1-N-[4-[[2-(2-morpholin-4-ylethylcarbamoyl)-1H-pyrrolo[2,3-b]pyridin-4-yl]oxy]phenyl]cyclopropane-1,1-dicarboxamide, 1-methyl-5-[2-(5-(trifluoromethyl)-1H-imidazol-2-yl]pyridin-4-yl]oxy-N-[4-(trifluoromethyl)phenyl]benzimidazol-2-amine, 6-[7-[(1-aminocyclopropyl)methoxy]-6-methoxyquinolin-4-yl]oxy-N-methylnaphthalene-1-carboxamide, or 2-dimethyl-6-[7-(2-morpholin-4-ylethoxy)quinolin-4-yl] oxy-1-benzofuran-3-carboxamide.
(4) The agent for reducing a side effect according to (1) above, wherein the kinase inhibitor is 4-[4-[[4-chloro-3-(trifluoromethyl)phenyl]carbamoylamino]phenoxy]-N-methylpyridine-2-carboxamide.
(5) The agent for reducing a side effect according to any one of (1) to (4) above, wherein the weight ratio between isoleucine, leucine and valine is 1 :(1.5 to 2.5):(0.8 to 1.7).
(6) The agent for reducing a side effect according to any one of (1) to (5) above, wherein the agent is administered to a liver cancer patient.
(7) The agent for reducing a side effect according to any one of (1) to (6) above, wherein the agent is administered to a patient having a liver stiffness stage of Child's classification A.
(8) An anticancer medicine, being an agent for use in anticancer therapy according to claim 12.
(9) The anticancer medicine according to (8) above, wherein the kinase inhibitor is 4-[4-[[4-chloro-3-(trifluoromethyl)phenyl]carbamoylamino]phenoxy]-N-methylpyridine-2-carboxamide, 4-[4-({[4-chloro-3-(trifluoromethyl)phenyl]carbamoyl}amino)-3-fluorophenoxy]-N-methylpyridine-2-carboxamide, N-(2-chloro-4-((6,7-dimethoxy-4-quinazolinyl)oxy)phenyl)-N'-propylurea, N-(3-[5-(2-aminopyrimidin-4-yl)-2-tert-butyl-1,3-thiazol-4-yl]-2-fluorophenyl]-2,6-difluorobenzenesulfonamide, 1-[1-[(2-aminopyridin-4-yl)methyl]indol-4-yl]-3-(5-bromo-2-methoxyphenyl)urea hydrochloride, N-[4-(3-amino-1H-indazol-4-yl)phenyl]-N'-(2-fluoro-5-methylphenyl)urea, 1-[2-chloro-4-(6,7-dimethoxyquinolin-4-yl)oxyphenyl]-3-(5-methyl-1,2-oxazol-3-yl)urea, 1-N'-[3-fluoro-4-[6-methoxy-7-(3-morpholin-4-ylpropoxy)quinolin-4-yl] oxyphenyl]-1-N-(4-fluorophenyl)cyclopropane-1,1-dicarboxamide, N-[[3-fluoro-4-[2-(1-methylimidazol-4-yl)thieno[3,2-b]pyridin-7-yl]oxyphenyl]carbamothioyl]-2-phenylacetamide, 1-N'-[2-fluoro-4-[2-[[4-(4-methylpiperazin-1-yl)piperidine-1-carbonyl]amino]pyridin-4-yl]oxyphenyl]-1-N-(4-fluorophenyl)cyclopropane-1,1-dicarboxamide, 4-[3-chloro-4-[[(cyclopropylamino)carbonyl]amino]phenoxy]-7-methoxy-monomethanesulfonate, 1-N'-(4-fluorophenyl)-1-N-(4-[[2-(2-morpholin-4-ylethylcarbamoyl)-1H-pyrrolo[2,3-b]pyridin-4-yl]oxy]phenyl]cyclopropane-1,1-dicarboxamide, 1-methyl-5-[2-[5-(trifluoromethyl)-1H-imidazol-2-yl]pyridin-4-yl]oxy-N-[4-(trifluoromethyl)phenyl]benzimidazol-2-amine, 6-[7-[(1-aminocyclopropyl)methoxy]-6-methoxyquinolin-4-yl]oxy-N-methylnaphthalene-1-carboxamide, or 2-dimethyl-6-[7-(2-morpholin-4-ylethoxy)quinolin-4-yl]oxy-1-benzofuran-3-carboxamide.
(10) The anticancer medicine according to (8) above, wherein the kinase inhibitor is 4-[4-[[4-chloro-3-(trifluoromethyl)phenyl]carbamoylamino]phenoxy]-N-methylpyridine-2-carboxamide.
(11) The anticancer medicine according to any one of (8) to (10) above, wherein the weight ratio between isoleucine, leucine and valine is 1:(1.5 to 2.5):(0.8 to 1.7).
(12) The anticancer medicine according to any one of (8) to (11) above, wherein the anticancer medicine is administered to a liver cancer patient.
(13) The anticancer medicine according to any one of (8) to (12) above, wherein the anticancer medicine is administered to a patient having a liver stiffness stage of Child's classification A.
(14) The anticancer medicine according to any one of (8) to (13) above, wherein the branched-chain amino acid or the salt thereof is taken together with the kinase inhibitor.

### EFFECTS OF THE INVENTION

The agent for reducing side effects according to the present invention can effectively reduce the side effects of kinase inhibitors such as sorafenib. As a result, by using the agent for reducing side effects of the present invention, or an anticancer medicine or medical kit containing this agent, in the treatment of diseases to which the various kinase inhibitors are applied, and particularly in the treatment of cancer, the quality of life (QOL) of the patient can be improved, and a superior therapeutic effect can be obtained.

### BRIEF DESCRIPTION OF THE DRAWINGS

FIG. 1 is a diagram illustrating the changes over time in the survival rate of each group in an Example 1.
FIG. 2 is a diagram illustrating the cell proliferation inhibitory effect of each group in an Example 2.
FIG. 3 is a diagram illustrating the antitumor action enhancement effect of each group in an Example 3.

### BEST MODE FOR CARRYING OUT THE INVENTION

### <Branched-Chain Amino Acid>

The isoleucine, leucine and valine which function as an active ingredient (branched-chain amino acid) of the present invention may each be used in the form of the L-isomer, the D-isomer or the DL-isomer, but the L-isomer or DL-isomer is preferable, and the L-isomer is the most desirable. Further, the isoleucine, leucine and valine may be used not only in free form, but also in the form of salts. Examples of these salts include acid addition salts and salts formed with bases, but the selection of a pharmaceutically acceptable salt of isoleucine, leucine or valine is preferable. Examples of acids which can be added to isoleucine, leucine and valine to form pharmaceutically acceptable salts include inorganic salts such as hydrogen chloride, hydrogen bromide, sulfuric acid and phosphoric salt, and organic acids such as acetic acid, lactic acid, citric acid, tartaric acid, maleic acid, fumaric acid and monomethyl sulfuric acid. Examples of pharmaceutically acceptable salts of isoleucine, leucine and valine with bases include salts formed with inorganic bases such as ammonia or the hydroxides or carbonates of metals such as sodium, potassium and calcium, and salts formed with organic bases such as ethylenediamine, propylenediamine, ethanolamine, monoalkylethanolamines, dialkylethanolamines, diethanolamine and triethanolamine.

### <Kinase Inhibitor>

In the present invention and the present description, the term "kinase inhibitor" includes inhibitors of cell surface kinases such as KIT, FLT-3, RET, VEGFR-1, VEGFR-2, VEGFR-3 and PDGFR-β, and inhibitors of intracellular kinases such as Raf.

Specific examples of the kinase inhibitors for which the side effects are reduced by using the side effect-reducing agent of the present invention include the compounds listed below.
4-[4-[[4-chloro-3-(trifluoromethyl)phenyl]carbamoylamino]phenoxy]-N-methylpyridine-2-carboxamide (sorafenib),
4-[4-({[4-chloro-3-(trifluoromethyl)phenyl]carbamoyl} amino)-3-fluorophenoxy]-N-methylpyridine-2-carboxamide,
N-(2-chloro-4-((6,7-dimethoxy-4-quinazolinyl)oxy)phenyl)-N'-propylurea,
N-[3-[5-(2-aminopyrimidin-4-yl)-2-tert-butyl-1,3-thiazol-4-yl]-2-fluorophenyl]-2,6-difluorobenzenesulfonamide, 1-[1-[(2-aminopyridin-4-yl)methyl]indol-4-yl]-3-(5-bromo-2-methoxyphenyl)urea hydrochloride,
N-[4-(3-amino-1H-indazol-4-yl)phenyl]-N'-(2-fluoro-5-methylphenyl)urea,
1-[2-chloro-4-(6,7-dimethoxyquinolin-4-yl)oxyphenyl]-3-(5-methyl-1,2-oxazol-3-yl)urea,
1-N'-[3-fluoro-4-[6-methoxy-7-(3-morpholin-4-ylpropoxy)quinolin-4-yl]oxyphenyl]-1-N-(4-fluorophenyl)cyclopropane-1,1-dicarboxamide,
N-[[3-fluoro-4-[2-(1-methylimidazol-4-yl)thieno[3,2-b]pyridin-7-yl]oxyphenyl]carbamothioyl]-2-phenylacetamide,
1-N'-[2-fluoro-4-[2-[[4-(4-methylpiperazin-1-yl)piperidine-1-carbonyl]amino]pyridin-4-yl]oxyphenyl]-1-N-(4-fluorophenyl)cyclopropane-1,1-dicarboxamide,
4-[3-chloro-4-[[(cyclopropylamino)carbonyl]amino]phenoxy]-7-methoxy-monomethanesulfonate,
1-N'-(4-fluorophenyl)-1-N-[4-[[2-(2-morpholin-4-ylethylcarbamoyl)-1H-pyrrolo[2,3-b]pyridin-4-yl]oxy]phenyl]cyclopropane-1,1-dicarboxamide,
1-methyl-5-[2-[5-(trifluoromethyl)-1H-imidazol-2-yl]pyridin-4-yl]oxy-N-[4-(trifluoromethyl)phenyl]benzimidazol-2-amine,
6-[7-[(1-aminocyclopropyl)methoxy]-6-methoxyquinolin-4-yl]oxy-N-methylnaphthalene-1-carboxamide, and
2-dimethyl-6-[7-(2-morpholin-4-ylethoxy)quinolin-4-yl]oxy-1-benzofuran-3-carboxamide.

In the present invention, the kinase inhibitor is preferably sorafenib.

The kinase inhibitor may be in the form of a pharmaceutically acceptable salt. Examples of acids which can form a pharmaceutically acceptable salt with the kinase inhibitor include the same acids as those mentioned above in relation to the branched-chain amino acid.

### <Agent for Reducing Side Effects>

The agent for reducing side effects according to the present invention contains the branched-chain amino acids isoleucine, leucine and valine or a salt thereof as an active ingredient, and has the property of reducing the side effects of kinase inhibitors.

The isoleucine, leucine and valine which represent the active ingredients of the present invention may be included in formulations either individually or in arbitrary combinations, or all three active ingredients may be included within a single formulation. When administered as separate formulations, the administration route and administration form may be the same or different for each formulation, and the timing of the administration of each formulation may also be either simultaneous or different. These decisions may be made as appropriate in accordance with the variety and effects of the drug being used in combination with the branched-chain amino acids.

The blend ratio (weight ratio) between the isoleucine, leucine and valine is preferably 1:(1 to 3):(0.5 to 2.0), more preferably 1:(1.5 to 2.5):(0.8 to 1.7), still more preferably 1:(1.5 to 2.5):(0.8 to 1.5), still more preferably 1 :(1.9 to 2.2):(1.1 to 1.3), and particularly preferably 1:2:1.2.

In the present invention, the "weight ratio" indicates the weight of each component within a formulation. For example, when isoleucine, leucine and valine are all included in a single formulation, the weight ratio indicates the amount of each active ingredient, whereas when the active ingredients are included across a plurality of formulations, either individually or in arbitrary combinations, the weight ratio describes the ratio of the weight of each active ingredient in each formulation.

Further, in the present invention, the actual dose ratio describes the ratio between the active ingredients per administration subject (namely, each patient) for a single dose or a daily dose. For example, when each of the active ingredients of isoleucine, leucine and valine are included in a single formulation, and that formulation is administered to a subject, the weight ratio is equal to the dose ratio. When the active ingredients are administered across a plurality of formulations, either individually or in arbitrary combinations, the ratio of the total of each active ingredient across each of the formulations administered in a single dose or across one day corresponds with the weight ratio.

Isoleucine, leucine and valine are already widely used in the fields of pharmaceuticals and foodstuffs, and their safety is well established. For example, the acute toxicity (LD₅₀) in a pharmaceutical composition of the present invention containing these amino acids in a ratio of 1:2:1.2 was more than 10 g/kg in the case of oral administration to mice.

The agent for reducing side effects according to the present invention can be formulated using normal methods to form solid formulation such as powders, granules, capsules, tablets or chewable tablets, liquid formulations such as solutions and syrups, or other formulations such as injections and sprays. These formulations can be administered by any appropriate administration method including oral administration, injection or local administration.

The agent for reducing side effects according to the present invention can be formulated by blending the branched-chain amino acids of the active ingredient with appropriate pharmaceutically acceptable carriers as required, including excipients, binders, lubricants, solvents, disintegrants, solubilizers, suspending agents, emulsifiers, isotonizing agents, stabilizers, pain relievers, preservatives, antioxidants, correctives and colorants.

Examples of the excipients include organic excipients, including sugars such as lactose, glucose and D-mannitol, starches, and celluloses such as crystalline cellulose, and inorganic excipients such as calcium carbonate and kaolin. Examples of the binders include α-starch, gelatin, gum arabic, methyl cellulose, carboxymethyl cellulose, sodium carboxymethylcellulose, crystalline cellulose, D-mannitol, trehalose, hydroxypropyl cellulose, hydroxypropylmethyl cellulose, polyvinylpyrrolidone and polyvinyl alcohol. Examples of the lubricants include stearic acid, fatty acid salts such as stearates, talc and silicates. Examples of the solvents include purified water and physiological saline solution. Examples of the disintegrants include low-substitution degree hydroxypropyl cellulose and chemically modified celluloses and starches. Examples of the solubilizers include polyethylene glycol, polypropylene glycol, trehalose, benzyl benzoate, ethanol, sodium carbonate, sodium citrate, sodium salicylate and sodium acetate. Examples of the suspending agents and emulsifiers include sodium laurate, gum arabic, gelatin, lecithin, glycerol monostearate, polyvinyl alcohol, polyvinylpyrrolidone, celluloses such as sodium carboxymethyl cellulose, polysorbates and polyoxyethylene hardened castor oil. Examples of the isotonizing agents include sodium chloride, potassium chloride, sugars, glycerol and urea. Examples of the stabilizers include polyethylene glycol, dextran sulfate sodium, and other amino acids. Examples of the pain relievers include glucose, calcium gluconate and procaine hydrochloride. Examples of the preservatives include paraoxybenzoate esters, chlorobutanol, benzyl alcohol, phenethyl alcohol, dehydroacetic acid and sorbic acid. Examples of the antioxidants include sulfite salts and ascorbic acid. Examples of the correctives include the types of sweeteners and flavorings typically used in the fields of pharmaceuticals and foodstuffs. Examples of the colorants include the types of coloring materials typically used in the fields of pharmaceuticals and foodstuffs.

By taking the agent for reducing side effects of the present invention, the side effects of kinase inhibitors are reduced. The agent for reducing side effects of the present invention is preferably taken to reduce the side effects of at least one of the group of kinase inhibitors specified above, and among this group of kinase inhibitors, the agent for reducing side effects is preferably taken to reduce the side effects of a kinase inhibitor taken as an anticancer drug, and more preferably taken to reduce the side effects of sorafenib. Because branched-chain amino acids themselves have an effect in inhibiting the development and progression of liver cancer (see Patent Document 2), the agent for reducing side effects according to the present invention is most preferably taken to reduce the side effects of sorafenib that has been administered as an anticancer drug for treating liver cancer.

In the present invention, the side effects treated are specifically hand-foot syndrome and bleeding (including gastrointestinal bleeding, respiratory tract bleeding, cerebral hemorrhaging, oral bleeding, nose bleeding, nail bed bleeding, hematoma and tumor hemorrhaging).

By taking the agent for reducing side effects according to the present invention in combination with a kinase inhibitor such as sorafenib, the side effects of the kinase inhibitor can be reduced. As a result, by taking the agent for reducing side effects according to the present invention, the QOL of a patient taking the kinase inhibitor can be improved. Further, in terms of the dose of the kinase inhibitor, even patients who, due to the severity of side effects, could conventionally only be administered with a small dose of the kinase inhibitor, are able to be more safely administered with a dose sufficient to achieve the desired drug efficacy. Administration of the agent for reducing side effects to patients having a liver stiffness stage of Child's classification A is particularly preferable. A patient with a Child's classification A describes a patient having a bilirubin level lower than 2.0 mg/dl and an albumin level higher than 3.5 g/dl, and is a patient having a comparatively good nutritional status with no symptoms of ascites or encephalopathy.

For example, when sorafenib is used as an anticancer drug, by taking the sorafenib in combination with the agent for reducing side effects of the present invention, the side-effects hand-foot syndrome and bleeding can be reduced dramatically, meaning that even for patients for whom the severity of these side effects would normally have resulted in the abandonment or temporary suspension of treatment, the sorafenib can be administered without any excessive loss in QOL. Furthermore, because of the lightness of the side effects, the form of administration can be selected more freely, allowing the dose to be increased or the administration period to be lengthened, and therefore the range of possible treatment plans can be broadened.

Besides the branched-chain amino acid or salt thereof, the agent for reducing side effects according to the present invention may also contain other medical components as active ingredients. Examples of these other medical components include anticancer drugs other than the aforementioned kinase inhibitors, and nutrients and the like such as vitamins.

### <Anticancer Medicine>

The anticancer medicine of the present invention contains a combination of all three branched-chain amino acids: isoleucine, leucine and valine or a salt thereof (namely, the agent for reducing side effects according to the present invention), and a kinase inhibitor, and is administered to a subject for the purpose of treating cancer. The anticancer medicine of the present invention may be a medicine in which the agent for reducing side effects according to the present invention and the kinase inhibitor are included in a single formulation, or a medicine in which the two components are combined as individual formulations.

The anticancer medicine of the present invention includes not only medicines used for treating cancer, but also medicines that inhibit the development or progression of cancer.

The kinase inhibitor can be formulated as any of a variety of formulations using conventional methods and pharmaceutically acceptable carriers and additives. Examples of the types of formulations and suitable pharmaceutically acceptable carriers, which may be used as required depending on the formulation, are the same as those listed above in relation to the agent for reducing side effects according to the present invention.

Any of the various commercially available kinase inhibitor formulations can be used as the kinase inhibitor. For example, sorafenib is available commercially as tablets of the tosylate salt under the brand name Nexavar (a registered trademark) (manufactured by Bayer Pharma AG).

There are no particular limitations on the patients used as treatment subjects for the anticancer medicine of the present invention, provided they are humans or animals suffering from cancer (malignant tumor). For example, the anticancer medicine may be used for treating primary cancer or metastatic cancer. Furthermore, the cancer may be early-stage cancer or advanced cancer. For example, when the kinase inhibitor is sorafenib, the anticancer medicine is preferably administered for the treatment of renal cell carcinoma and hepatocellular carcinoma, and is most preferably administered to patients having unresectable hepatocellular carcinoma and having a liver stiffness stage of Child's classification A.

### <Administration Method, Dose>

In the agent for reducing side effects and the anticancer medicine according to the present invention, the administered dose (intake) of the branched-chain amino acids varies depending on the condition and age of the patient and the administration method and the like, but is typically equivalent to a dose, per person per day, of 0.5 to 30.0 g of isoleucine, 1.0 to 60.0 g of leucine, and 0.5 to 30.0 g of valine. In the case of a typical adult, the dose per person per day is preferably 2.0 to 10.0 g of isoleucine, 3.0 to 20.0 g of leucine, and 2.0 to 10.0 g of valine, and more preferably 2.5 to 3.5 g of isoleucine, 5.0 to 7.0 g of leucine, and 3.0 to 4.0 g of valine. The administered dose per adult per day, calculated as the combined total of all three types of branched-chain amino acid, is typically approximately 2.0 to 50.0 g, which if required, may be divided into 1 to 6 administered portions, and preferably 1 to 3 portions.

The amount of branched-chain amino acids in a single administered dose is typically approximately 0.5 to 50.0 g, preferably approximately 1.0 to 20.0 g, and more preferably approximately 2.0 to 6.0 g.

There are no particular limitations on the administered dose, administration period, administration schedule or administration route or the like of the kinase inhibitor, provided that the efficacy of the kinase inhibitor manifests satisfactorily. For example, the dose for each of the kinase inhibitors specified above, regardless of the administration route, may be within a range from 0.01 to 200 mg/kg (bodyweight) per day. When the kinase inhibitor is sorafenib, an adult is preferably orally administered with 400 mg of sorafenib per dose, either every second day, or 1 or 2 times per day.

In the present invention, the branched-chain amino acids and the kinase inhibitor may be administered as separate formulations, using either the same or different forms of administration. Alternatively, they may be administered as a combination drug containing both the branched-chain amino acids and the kinase inhibitor.

When the branched-chain amino acids and the kinase inhibitor are separate formulations, there are no particular limitations on the form of administration used, provided that the branched-chain amino acids and the kinase inhibitor both exist in vivo at the same time, and for example, the branched-chain amino acids may be administered at the same time as the kinase inhibitor, or administered either before or after the kinase inhibitor. The administration method and doses used in the case of joint administration may be selected appropriately in accordance with the type of drug being used and the effects of the drug.

When the branched-chain amino acids and the kinase inhibitor are administered with a time difference therebetween, the time difference may vary depending on the administered active ingredients, the dosage form and the administration method, and for example in the case where the branched-chain amino acids are administered first, the kinase inhibitor is typically administered within a period of 5 minutes to 14 days, and more preferably within a period of 10 minutes to 7 days, from the administration of the branched-chain amino acids. When the kinase inhibitor is administered first, the branched-chain amino acids are typically administered within a period of 5 minutes to 120 hours, and more preferably within a period of 10 minutes to 80 hours, from the administration of the kinase inhibitor.

When calculating the dose (intake) of the active ingredient branched-chain amino acids used in the present invention, the numerical range described above is determined on the basis of the amount of the active ingredient within the medicine used for the purpose of reducing the side effects of the kinase inhibitor, and therefore if branched-chain amino acids are also taken or administered for other purposes, such as within the normal daily diet or for the treatment of a different disease, these amounts need not be included within the above calculations. For example, the amount of branched-chain amino acids consumed per day as part of a normal diet need not be subtracted from the daily administration dose of the active ingredient in the present invention.

### <Medical Kit>

The branched-chain amino acids isoleucine, leucine and valine or a salt thereof (namely, the agent for reducing side effects according to the present invention) may be combined with an effective amount of a kinase inhibitor formulation in the form of individual formulations, and these formulations may then be packaged together to form a medical kit.

The medical kit preferably also contains documentation disclosing that the agent for reducing side effects according to the present invention can be used, or should be used, for reducing the side effects of the kinase inhibitor. This documentation may be added to the kit in the form of a document printed on paper or the like, may be printed directly onto the container containing the agent for reducing side effects according to the present invention, or may be printed directly onto the box or plastic bag or the like containing both the agent for reducing side effects according to the present invention and the kinase inhibitor. Instead of printing directly to the container or box or the like, a printed seal may also be affixed.

### EXAMPLES

The present invention is described below in further detail using a series of examples and the like, but the present invention is in no way limited by these examples.

### [Example 1]

Using CDAA (choline-deficient L-amino acid defined) diet-induced liver cancer model rats, the effects of the combined intake of sorafenib and branched-chain amino acids (BCAA) were investigated. The CDAA diet used a commercially available choline-deficient diet (#518753: Choline deficient and iron supplemented L-amino acid defined rat diet, manufactured by Dyets Inc.).

Six-week old Fischer 344 rats were divided into a CDAA diet intake group, a sorafenib only intake group, and a BCAA combined intake group (N=15 for each group), and the test diet was provided freely. For the CDAA diet intake group, only the CDAA diet was supplied as the test diet. For the sorafenib only intake group, a CDAA diet mixed with an amount of sorafenib tosylate tablets (Nexavar (a registered trademark) tablets 200 mg, manufactured by Bayer Pharma AG) sufficient to provide an intake of sorafenib of 16 mg/kg/day (800 mg/50 kg/day) (hereafter referred to as the "CDAA/sorafenib diet") was supplied. For the BCAA combined intake group, a diet prepared by mixing the CDAA/sorafenib diet supplied to the sorafenib only intake group with 2.5% by mass of a BCAA formulation (Livact (a registered trademark) granules, manufactured by Ajinomoto Co., Inc.) (hereafter referred to as the "CDAA/sorafenib/BCAA diet") was supplied. The weight ratio between isoleucine, leucine and valine in the BCAA formulation was 1:2:1.2 (isoleucine:0.952 g, leucine: 1.904 g, and valine: 1.144 g).

Each individual was autopsied either at the time of death or after 56 weeks from the start of the test (from supply of the test diet), and the effect of treatment continuation was investigated on the basis of the hepatic fibrosis inhibitory effect, the hepatocarcinogenesis rate and the survival rate.

Further, the presence or absence of hepatic fibrosis was determined on the basis of the result of Azan staining of a liver tissue section, and the result of measuring the concentration of HYP (hydroxyproline) within the liver. The presence or absence of hepatocarcinogenesis was evaluated by immunostaining a tissue section of the liver with an antibody to the precancerous marker GST-P (glutathione S-transferase placental form), and inspecting the tissue section for the presence of staining.

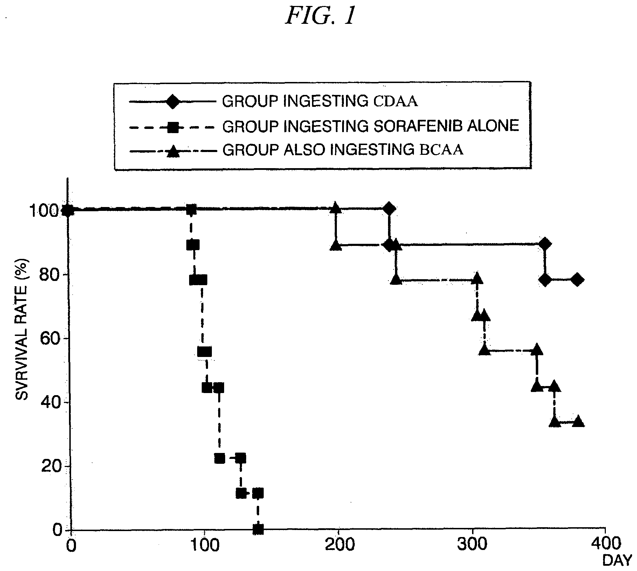

The changes over time in the survival rates for each group are shown in FIG. 1. In the CDAA diet intake group and the BCAA combined intake group, all of the rats were alive 20 weeks after starting the test, whereas in the sorafenib only intake group, all the individuals died 16 to 20 weeks after starting the test, displaying cutaneous symptoms or whole body bleeding, indicating a significant increase in the death rate. In other words, in the BCAA combined intake group, the survival rate improved dramatically compared with the sorafenib only intake group. Further, compared with the CDAA diet intake group, the number of rats in which hepatic fibrosis or precancerous lesions were observed was fewer in the sorafenib only intake group and the BCAA combined intake group, confirming that the onset of liver cancer was inhibited in these two groups. Furthermore, in the sorafenib only intake group, redness was noted not only on the four legs, but also on the trunk and the skin, and a tendency for bleeding to occur in the gastrointestinal tract, including the anal region, was also observed. Moreover, a loss of appetite due to gastrostenosis was also noted. In contrast, in the BCAA combined intake group, the symptoms of redness, bleeding and loss of appetite were all improved significantly.

Based on these results, it was clear that in a choline-deficient L-amino acid defined diet-induced hepatic fibrosis and liver cancer rat model, by jointly administering sorafenib and BCAA, a hepatic fibrosis inhibitory effect and a liver precancerous lesion inhibitory effect were obtained, and the survival rate and level of side effects were improved significantly compared with the case of administering only sorafenib, enabling continued treatment to be performed safely.

### [Example 2]

### (Cell Proliferation Inhibitory Effect)

Each of the wells of a 96 well plate was inoculated with 6×10³ cell/well of Huh7 cells, and on the following day, the medium was exchanged with an LC medium (containing 10% FBS), sorafenib of concentration 0, 1, 2, 4 or 8 µM containing 2 mM of BCAA was added, and incubation was performed for 48 hours. Samples containing no added BCAA were also incubated for 48 hours as controls.

After 48 hours, the medium was removed, 4% paraformaldehyde was added to each well and fixation was performed for 15 minutes. Following removal of the paraformaldehyde, a 5% Hoechst solution was added to each well, and left to stand for one minute. The 5% Hoechst solution was then removed, and 200 µl of a PBS solution was added to each well.

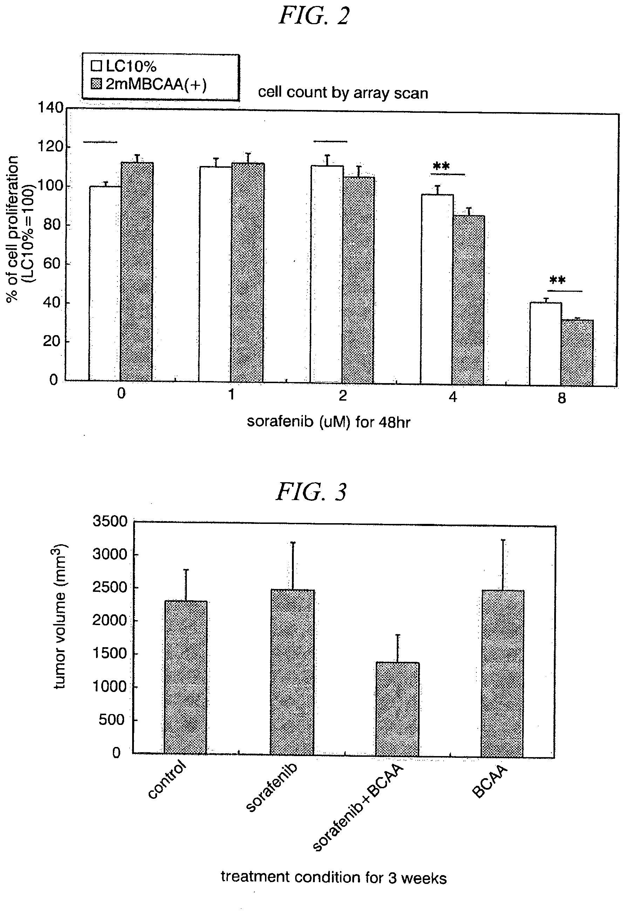

The plate was analyzed for cell count using an array scan, and the cell count in each group was calculated as a % of cell proliferation relative to a value of 100 for the cell count of the control (LC 10%). The results are shown in FIG. 2.

As is evident from FIG. 2, in the sorafenib + BCAA groups, and particularly in the cases where combined stimulation with BCAA was used in the 2, 4 and 8 µM stimulation with sorafenib, a significant cell proliferation inhibitory effect was observed compared with the case of stimulation with only sorafenib.

### [Example 3]

### (Antitumor Action Enhancement Effect)

Huh7 cells were implanted subcutaneously in BALB/c nude mice in an amount of 1 × 10⁷ cell/mouse.

After one week, the mice were split into groups based on tumor diameter, and the groups were subjected to 2 weeks of 5 mg/kg sorafenib oral administration ± food supply containing 3% BCAA (with the sorafenib administered 5 days/week).

In the third week of administration, the sorafenib dose was increased from 5 mg/kg to 30 mg/kg, and oral administration was continued for 5 days. During this time, supply of the feed containing 3% BCAA was continued.

Three weeks after the start of administration, the tumor diameter of each group was measured, and the average value + SE for the tumor volume was calculated. The tumor volume was calculated as tumor major axis/2 × (minor axis)².

The results are shown in FIG. 3.

As is evident from the results of FIG. 3, in the sorafenib+BCAA group, the degree of shrinkage in the tumor volume was large compared with the control group, and a tendency for an enhanced antitumor effect was observed as a result of combined administration of the two components.

In the sorafenib only group and the BCAA only group, the degree of shrinkage in the tumor volume was unchanged from that of the control group.

### INDUSTRIAL APPLICABILITY

The agent for reducing side effects according to the present invention can effectively reduce the specified side effects of kinase inhibitors such as sorafenib. As a result, the agent for reducing side effects of the present invention, and an anticancer medicine and medical kit containing this agent, are ideal for the medical treatment of diseases to which the various kinase inhibitors are applied, and particularly for the treatment of cancers that are treated using sorafenib.

## Claims

1. A branched-chain amino acid, or salt thereof, which comprises all three branched-chain amino acids: isoleucine, leucine, and valine, for use in reducing a side effect of a kinase inhibitor, wherein the side effect is at least one of: hand-foot syndrome and bleeding.

2. The branched-chain amino acid, or salt thereof, for use according to claim 1, wherein the kinase inhibitor is:
4-[4-[[4-chloro-3-(trifluoromethyl)phenyl]carbamoylamino]phenoxy]-N-methylpyridine-2-carboxamide,
4-[4-({[4-chloro-3-(trifluoromethyl)phenyl]carbamoyl}amino)-3-fluorophenoxy]-N-methylpyridine-2-carboxamide,
N-(2-chloro-4-((6,7-dimethoxy-4-quinazolinyl)oxy)phenyl)-N'-propylurea, N-[3-[5-(2-aminopyrimidin-4-yl)-2-tert-butyl-1,3-thiazol-4-yl]-2-fluorophenyl]-2,6-difluorobenzenesulfonamide,
1-[1-[(2-aminopyridin-4-yl)methyl]indol-4-yl]-3-(5-bromo-2-methoxyphenyl)urea hydrochloride,
N-[4-(3-amino-1H-indazol-4-yl)phenyl]-N'-(2-fluoro-5-methylphenyl)urea, 1-[2-chloro-4-(6,7-dimethoxyquinolin-4-yl)oxyphenyl]-3-(5-methyl-1,2-oxazol-3-yl)urea,
1 -N'-[3-fluoro-4-[6-methoxy-7-(3-morpholin-4-ylpropoxy)quinolin-4-yl]oxyphenyl]-1-N-(4-fluorophenyl)cyclopropane-1,1-dicarboxamide,
N-[[3-fluoro-4-[2-(1-methylimidazol-4-yl)thieno[3,2-b]pyridin-7-yl]oxyphenyl]carbamothioyl]-2-phenylacetamide,
1 -N'- [2-fluoro-4- [2-[[4-(4-methylpiperazin-1-yl)piperidine-1-carbonyl]amino]pyridin-4-yl]oxyphenyl]-1-N-(4-fluorophenyl)cyclopropane-1,1-dicarboxamide,
4-[3-chloro-4-[[(cyclopropylamino)carbonyl]amino]phenoxy]-7-methoxy-monomethanesulfonate,
1-N'-(4-fluorophenyl)-1-N-[4-[[2-(2-morpholin-4-ylethylcarbamoyl)-1H-pyrrolo [2,3-b]pyridin-4-yl]oxy]phenyl]cyclopropane-1,1 -dicarboxamide, 1-methyl-5-[2-[5-(trifluoromethyl)-1H-imidazol-2-yl]pyridin-4-yl]oxy-N-[4-(trifluoromethyl)phenyl]benzimidazol-2-amine,
6-[7-[(1-aminocyclopropyl)methoxy]-6-methoxyquinolin-4-yl]oxy-N-methylnaphthalene-1-carboxamide,
or 2-dimethyl-6-[7-(2-morpholin-4-ylethoxy)quinolin-4-yl]oxy-1-benzofuran-3-carboxamide.

3. The branched-chain amino acid, or salt thereof, for use according to claim 1, wherein the kinase inhibitor is 4-[4-[[4-chloro-3-(trifluoromethyl)phenyl]carbamoylamino]phenoxy]-N-methylpyridine-2-carboxamide.

4. The branched-chain amino acid, or salt thereof, for use according to to claim 1, wherein the weight ratio between isoleucine, leucine and valine is 1:(1.5 to 2.5):(0.8 to 1.7).

5. The branched-chain amino acid, or salt thereof, of any one of claims 1 to 4 for use according to any one of claims 1 to 4 in a liver cancer patient.

6. The branched-chain amino acid, or salt thereof, of any one of claims 1 to 5 for use according to any one of claims 1 to 5 in a patient having a liver stiffness stage of Child's classification A.

7. The branched-chain amino acid, or salt thereof, for use according to any one of claims 1 to 6, wherein the branched-chain amino acid, or salt thereof, is administered at the same time as the kinase inhibitor.

8. The branched-chain amino acid, or salt thereof, for use according to any one of claims 1 to 6, wherein the branched-chain amino acid, or salt thereof, is administered before, or after, administration of the kinase inhibitor.

9. A pharmaceutical composition for use in reducing a side effect of a kinase inhibitor, wherein the pharmaceutical composition comprises the branched-chain amino acid, or salt thereof, according to any one of claims 1 to 6, and wherein the side effect is at least one of: hand-foot syndrome and bleeding.

10. A food composition for use in reducing a side effect of the kinase inhibitor, wherein the food composition comprises the branched-chain amino acid, or salt thereof, according to any one of claims 1 to 6, and wherein the side effect is at least one of hand-foot syndrome and bleeding.

11. The food composition for use according to claim 10, wherein the food composition is contained in a container.

12. An agent for use in anticancer therapy comprising:
the branched-chain amino acid, or salt thereof, according to any one of claims 1 to 6, and
a kinase inhibitor, wherein
the branched-chain amino acid, or salt thereof, and the kinase inhibitor are arranged for administration as a combination drug, or for separate, simultaneous or sequential administration; and wherein
the branched-chain amino acid is for use in reducing a side effect of the kinase inhibitor wherein the side effect is at least one of: hand-foot syndrome and bleeding.

13. The agent for use in anticancer therapy according to claim 12, wherein the kinase inhibitor is
4-[4-[[4-chloro-3-(trifluoromethyl)phenyl]carbamoylamino]phenoxy]-N-methylpyridine-2-carboxamide,
4-[4-({[4-chloro-3-(trifluoromethyl)phenyl]carbamoyl}amino)-3-fluorophenoxy]-N-methylpyridine-2-carboxamide,
N-(2-chloro-4-((6,7-dimethoxy-4-quinazolinyl)oxy)phenyl)-N'-propylurea,
N-[3-[5-(2-aminopyrimidin-4-yl)-2-tert-butyl-1,3-thiazol-4-yl]-2-fluorophenyl]-2,6-difluorobenzenesulfonamide,
1-[1-[(2-aminopyridin-4-yl)methyl]indol-4-yl]-3-(5-bromo-2-methoxyphenyl)urea hydrochloride,
N-[4-(3-amino-1H-indazol-4-yl)phenyl]-N'-(2-fluoro-5-methylphenyl)urea,
1-[2-chloro-4-(6,7-dimethoxyquinolin-4-yl)oxyphenyl]-3-(5-methyl-1,2-oxazol-3-yl)urea,
1-N'-[3-fluoro-4-[6-methoxy-7-(3-morpholin-4-ylpropoxy)quinolin-4-yl]oxyphenyl]-1-N-(4-fluorophenyl)cyclopropane-1,1-dicarboxamide,
N-[[3-fluoro-4-[2-(1-methylimidazol-4-yl)thieno[3,2-b]pyridin-7-yl]oxyphenyl]carbamothioyl]-2-phenylacetamide,
1 -N'-[2-fluoro-4- [2-[[4-(4-methylpiperazin-1-yl)piperidine-1-carbonyl]amino]pyridin-4-yl]oxyphenyl]-1-N-(4-fluorophenyl)cyclopropane-1,1-dicarboxamide,
4-[3-chloro-4-[[(cyclopropylamino)carbonyl]amino]phenoxy]-7-methoxy-monomethanesulfonate,
1-N'-(4-fluorophenyl)-1-N-[4-[[2-(2-morpholin-4-ylethylcarbamoyl)-1H-pyrrolo[2,3-b]pyridin-4-yl]oxy]phenyl]cyclopropane-1,1 -dicarboxamide,
1-methyl-5-[2-[5-(trifluoromethyl)-1H-imidazol-2-yl]pyridin-4-yl]oxy-N-[4-(trifluoromethyl)phenyl]benzimidazol-2-amine,
6-[7-[(1-aminocyclopropyl)methoxy]-6-methoxyquinolin-4-yl]oxy-N-methylnaphthalene-1 -carboxamide,
or 2-dimethyl-6-[7-(2-morpholin-4-ylethoxy)quinolin-4-yl]oxy-1-benzofuran-3-carboxamide.

## Patentansprüche

1. Verzweigte Aminosäure oder Salz davon, die/das alle drei verzweigten Aminosäuren Isoleucin, Leucin und Valin umfasst, zur Verwendung zur Verringerung einer Nebenwirkung eines Kinaseinhibitors, wobei die Nebenwirkung zumindest eines von Hand-Fuß-Syndrom und Bluten ist.

2. Verzweigte Aminosäure oder Salz davon zur Verwendung nach Anspruch 1, wobei der Kinaseinhibitor Folgendes ist:
4-[4-[[4-Chlor-3-(trifluormethyl)phenyl]carbamoylamino]phenoxy]-N-methylpyridin-2-carbox-amid,
4-[4-({[4-Chlor-3-(trifluormethyl)phenyl]carbamoyl}amino)-3-fluorphenoxy]-N-methylpyridin-2-carboxamid,
N-(2-Chlor-4-((6,7-dimethoxy-4-chinazolinyl)oxy)phenyl)-N'-propylharnstoff,
N-[3-[5-(2-Aminopyrimidin-4-yl)-2-tert-butyl-1,3-thiazol-4-yl]-2-fluorphenyl]-2,6-difluorbenzol-sulfonamid,
1-[1-[(2-Aminopyridin-4-yl)methyl]indol-4-yl]-3-(5-brom-2-methoxyphenyl)harnstoffhydrochlo-rid,
N-[4-(3-Amino-1H-indazol-4-yl)phenyl]-N'-(2-fluor-5-methylphenyl)harnstoff,
1-[2-Chlor-4-(6,7-dimethoxychinolin-4-yl)oxyphenyl]-3-(5-methyl-1,2-oxazol-3-yl)harnstoff,
1-N'-[3-Fluor-4-[6-methoxy-7-(3-morpholin-4-ylpropoxy)chinolin-4-yl]oxyphenyl]-1-N-(4-fluorphenyl)cyclopropan-1,1-dicarboxamid,
N-[[3-Fluor-4-[2-(1-methylimidazol-4-yl)thieno[3,2-b]pyridin-7-yl]oxyphenyl]carbamothioyl]-2-phenylacetamid,
1-N'-[2-Fluor-4-[2-[[4-(4-methylpiperazin-1-yl)piperidin-1-carbonyl]amino]pyridin-4-yl]oxyphe-nyl]-1-N-(4-fluorphenyl)cyclopropan-1,1-dicarboxamid,
4-[3-Chlor-4-[[(cyclopropylamino)carbonyl]amino]phenoxy]-7-methoxymonomethansulfonat,
1-N'-(4-Fluorphenyl)-1-N-[4-[[2-(2-morpholin-4-ylethylcarbamoyl)-1H-pyrrolo[2,3-b]pyridin-4-yl]oxy]phenyl]cyclopropan-1,1-dicarboxamid,
1-Methyl-5-[2-[5-(trifluormethyl)-1H-imidazol-2-yl]pyridin-4-yl]oxy-N-[4-(trifluormethyl)phenyl]-benzimidazol-2-amin,
6-[7-[(1-Aminocyclopropyl)methoxy]-6-methoxychinolin-4-yl]oxy-N-methylnaphthalin-1-car-boxamid oder
2-Dimethyl-6-[7-(2-morpholin-4-ylethoxy)chinolin-4-yl]oxy-1-benzofuran-3-carboxamid.

3. Verzweigte Aminosäure oder Salz davon zur Verwendung nach Anspruch 1,
wobei der Kinaseinhibitor 4-[4-[[4-Chlor-3-(trifluormethyl)phenyl]carbamoylamino]phenoxy]-N-methylpyridin-2-carboxamid ist.

4. Verzweigte Aminosäure oder Salz davon zur Verwendung nach Anspruch 1,
wobei das Gewichtsverhältnis zwischen Isoleucin, Leucin und Valin 1:(1,5 bis 2,5):(0,8 bis 1,7) beträgt.

5. Verzweigte Aminosäure oder Salz davon nach einem der Ansprüche 1 bis 4 zur Verwendung nach einem der Ansprüche 1 bis 4 bei einem Leberkrebspatienten.

6. Verzweigte Aminosäure oder Salz davon nach einem der Ansprüche 1 bis 5 zur Verwendung nach einem der Ansprüche 1 bis 5 bei einem Patienten, der ein Lebersteifigkeitsstadium der Child-Klassifikation A aufweist.

7. Verzweigte Aminosäure oder Salz davon zur Verwendung nach einem der Ansprüche 1 bis 6, wobei die verzweigte Aminosäure oder das Salz davon gleichzeitig mit dem Kinaseinhibitor verabreicht wird.

8. Verzweigte Aminosäure oder Salz davon zur Verwendung nach einem der Ansprüche 1 bis 6, wobei die verzweigte Aminosäure oder das Salz davon vor oder nach der Verabreichung des Kinaseinhibitors verabreicht wird.

9. Pharmazeutische Zusammensetzung zur Verwendung zur Verringerung einer Nebenwirkung eines Kinaseinhibitors, wobei die pharmazeutische Zusammensetzung die verzweigte Aminosäure oder das Salz davon nach einem der Ansprüche 1 bis 6 umfasst und wobei die Nebenwirkung zumindest eines von Hand-Fuß-Syndrom und Bluten ist.

10. Nahrungsmittelzusammensetzung zur Verwendung zur Verringerung einer Nebenwirkung eines Kinaseinhibitors, wobei die Nahrungsmittelzusammensetzung die verzweigte Aminosäure oder das Salz davon nach einem der Ansprüche 1 bis 6 umfasst und wobei die Nebenwirkung zumindest eines von Hand-Fuß-Syndrom und Bluten ist.

11. Nahrungsmittelzusammensetzung zur Verwendung nach Anspruch 10, wobei die Nahrungsmittelzusammensetzung in einem Behältnis enthalten ist.

12. Mittel zur Verwendung in der Antikrebstherapie, Folgendes umfassend:
die verzweigte Aminosäure oder das Salz davon nach einem der Ansprüche 1 bis 6 und
einen Kinaseinhibitor, wobei
die verzweigte Aminosäure oder das Salz davon und der Kinaseinhibitor zur Verabreichung als ein Kombinationsarzneimittel oder zur separaten, simultanen oder sequenziellen Verabreichung zusammengestellt sind; und wobei
die verzweigte Aminosäure zur Verwendung zur Verringerung einer Nebenwirkung des Kinaseinhibitors dient, wobei die Nebenwirkung zumindest eines von Hand-Fuß-Syndrom und Bluten ist.

13. Mittel zur Verwendung in der Antikrebstherapie nach Anspruch 12, wobei der Kinaseinhibitor Folgendes ist:
4-[4-[[4-Chlor-3-(trifluormethyl)phenyl]carbamoylamino]phenoxy]-N-methylpyridin-2-carbox-amid,
4-[4-({[4-Chlor-3-(trifluormethyl)phenyl]carbamoyl}amino)-3-fluorphenoxy]-N-methylpyridin-2-carboxamid,
N-(2-Chlor-4-((6,7-dimethoxy-4-chinazolinyl)oxy)phenyl)-N'-propylharnstoff,
N-[3-[5-(2-Aminopyrimidin-4-yl)-2-tert-butyl-1,3-thiazol-4-yl]-2-fluorphenyl]-2,6-difluorbenzol-sulfonamid,
1-[1-[(2-Aminopyridin-4-yl)methyl]indol-4-yl]-3-(5-brom-2-methoxyphenyl)harnstoffhydrochlo-rid,
N-[4-(3-Amino-1H-indazol-4-yl)phenyl]-N'-(2-fluor-5-methylphenyl)harnstoff,
1-[2-Chlor-4-(6,7-dimethoxychinolin-4-yl)oxyphenyl]-3-(5-methyl-1,2-oxazol-3-yl)harnstoff,
1-N'-[3-Fluor-4-[6-methoxy-7-(3-morpholin-4-ylpropoxy)chinolin-4-yl]oxyphenyl]-1-N-(4-fluorphenyl)cyclopropan-1,1-dicarboxamid,
N-[[3-Fluor-4-[2-(1-methylimidazol-4-yl)thieno[3,2-b]pyridin-7-yl]oxyphenyl]carbamothioyl]-2-phenylacetamid,
1-N'-[2-Fluor-4-[2-[[4-(4-methylpiperazin-1-yl)piperidin-1-carbonyl]amino]pyridin-4-yl]oxyphe-nyl]-1-N-(4-fluorphenyl)cyclopropan-1,1-dicarboxamid,
4-[3-Chlor-4-[[(cyclopropylamino)carbonyl]amino]phenoxy]-7-methoxymonomethansulfonat,
1-N'-(4-Fluorphenyl)-1-N-[4-[[2-(2-morpholin-4-ylethylcarbamoyl)-1H-pyrrolo[2,3-b]pyridin-4-yl]oxy]phenyl]cyclopropan-1,1-dicarboxamid,
1- Methyl-5-[2-[5-(trifluormethyl)-1H-imidazol-2-yl]pyridin-4-yl]oxy-N-[4-(trifluormethyl)phenyl]-benzimidazol-2-amin,
6-[7-[(1-Aminocyclopropyl)methoxy]-6-methoxychinolin-4-yl]oxy-N-methylnaphthalin-1-car-boxamid oder
2-Dimethyl-6-[7-(2-morpholin-4-ylethoxy)chinolin-4-yl]oxy-1-benzofuran-3-carboxamid.

## Revendications

1. Acide aminé à chaîne ramifiée, ou un sel de celui-ci, qui comprend les trois acides aminés à chaîne ramifiée ensemble : l'isoleucine, la leucine et la valine, pour une utilisation dans la réduction d'un effet secondaire d'un inhibiteur de kinase, dans lequel l'effet secondaire est au moins un effet choisi parmi le syndrome main-pied et un saignement.

2. Acide aminé à chaine ramifiée, ou un sel de celui-ci, pour une utilisation selon la revendication 1, dans lequel l'inhibiteur de kinase est :
le 4-[4-[[4-chloro-3-(trifluorométhyl)phényl]carbamoyl-amino]phénoxy]-N-méthylpyridine-2-carboxamide,
le 4-[4-({[4-chloro-3-(trifluorométhyl)phényl]carbamoyl}-amino)-3-fluorophénoxy]-N-méthylpyridine-2-carboxamide,
la N-(2-chloro-4-((6,7-diméthoxy-4-quinazolinyl)oxy)-phényl)-N'-propylurée,
le N-[3-[5-(2-aminopyrimidin-4-yl)-2-tert-butyl-1,3-thiazol-4-yl]-2-fluorophényl]-2,6-difluorobenzenesulfonamide,
le chlorhydrate de 1-[1-[(2-aminopyridin-4-yl)méthyl]-indol-4-yl]-3-(5-bromo-2-méthoxyphényl)urée,
la N-[4-(3-amino-1H-indazol-4-yl)phényl]-N'-(2-fluoro-5-méthylphényl)urée,
la 1-[2-chloro-4-(6,7-diméthoxyquinoléin-4-yl)oxyphényl]-3-(5-méthyl-1,2-oxazol-3-yl)urée,
le 1-N'-[3-fluoro-4-[6-méthoxy-7-(3-morpholin-4-yl-propoxy)quinoléin-4-yl]oxyphényl]-1-N-(4-fluorophényl)-cyclopropane-1,1-dicarboxamide,
le N-[[3-fluoro-4-[2-(1-méthylimidazol-4-yl)thiéno[3,2-b]pyridin-7-yl]oxyphényl]carbamothioyl]-2-phénylacétamide,
le 1-N'-[2-fluoro-4-[2-[[4-(4-méthylpipérazin-1-yl)-pipéridine-1-carbonyl]amino]pyridin-4-yl]oxyphényl]-1-N-(4-fluorophényl)cyclopropane-1,1-dicarboxamide,
le 4-[3-chloro-4-[[(cyclopropylamino)carbonyl]amino]-phénoxy]-7-méthoxy-monométhanesulfonate,
le 1-N'-(4-fluorophényl)-1-N-[4-[[2-(2-morpholin-4-yl-éthylcarbamoyl)-1H-pyrrolo[2,3-b]pyridin-4-yl]oxy]phényl]-cyclopropane-1,1-dicarboxamide,
la 1-méthyl-5-[2-[5-(trifluorométhyl)-1H-imidazol-2-yl]-pyridin-4-yl]oxy-N-[4-(trifluorométhyl)phényl]-benzimidazol-2-amine,
le 6-[7-[(1-aminocyclopropyl)méthoxy]-6-méthoxyquinoléin-4-yl]oxy-N-méthylnaphtalène-1-carboxamide,
ou le 2-diméthyl-6-[7-(2-morpholin-4-yl-éthoxy)quinoléin-4-yl]oxy-1-benzofuran-3-carboxamide.

3. Acide aminé à chaîne ramifiée, ou un sel de celui-ci, pour une utilisation selon la revendication 1, dans lequel l'inhibiteur de kinase est le 4-[4-[[4-chloro-3-(trifluorométhyl)phényl]carbamoylamino]phénoxy]-N-méthylpyridine-2-carboxamide.

4. Acide aminé à chaîne ramifiée, ou un sel de celui-ci, pour une utilisation selon la revendications 1, dans lequel le rapport pondéral entre l'isoleucine, la leucine et la valine est 1:(1,5 à 2,5):(0,8 à 1,7).

5. Acide aminé à chaîne ramifiée, ou un sel de celui-ci, pour une utilisation selon l'une quelconque des revendications 1 à 4 chez un patient atteint d'un cancer du foie.

6. Acide aminé à chaîne ramifiée, ou un sel de celui-ci, pour une utilisation selon l'une quelconque des revendications 1 à 5 chez un patient présentant un stade de raideur du foie selon la classification de Child A.

7. Acide aminé à chaîne ramifiée, ou un sel de celui-ci, pour une utilisation selon l'une quelconque des revendications 1 à 6, dans lequel l'acide aminé à chaîne ramifiée, ou un sel de celui-ci, est administré en même temps que l'inhibiteur de kinase.

8. Acide aminé à chaîne ramifiée, ou un sel de celui-ci, pour une utilisation selon l'une quelconque des revendications 1 à 6, dans lequel l'acide aminé à chaîne ramifiée, ou un sel de celui-ci, est administré avant ou après l'administration de l'inhibiteur de kinase.

9. Composition pharmaceutique destinée à une utilisation pour réduire un effet secondaire d'un inhibiteur de kinase, la composition pharmaceutique comprenant l'acide aminé à chaîne ramifiée, ou un sel de celui-ci, selon l'une quelconque des revendications 1 à 6, et l'effet secondaire étant au moins un effet choisi parmi le syndrome main-pied et un saignement.

10. Composition alimentaire destinée à une utilisation pour réduire un effet secondaire de l'inhibiteur de kinase, la composition alimentaire comprenant l'acide aminé à chaîne ramifiée, ou un sel de celui-ci, selon l'une quelconque des revendications 1 à 6, et l'effet secondaire étant au moins un effet choisi parmi le syndrome main-pied et un saignement.

11. Composition alimentaire pour une utilisation selon la revendication 10, la composition alimentaire étant contenue dans un récipient.

12. Agent pour une utilisation en thérapie anticancéreuse comprenant :
l'acide aminé à chaîne ramifiée, ou un sel de celui-ci, selon l'une quelconque des revendications 1 à 8, et
un inhibiteur de kinase,
l'acide aminé à chaîne ramifiée, ou un sel de celui-ci, et l'inhibiteur de kinase étant prévus pour une administration sous forme d'association médicamenteuse, ou pour une administration séparée, simultanée ou séquentielle ; et dans lequel l'acide aminé à chaîne ramifiée est destiné à une utilisation dans la réduction d'un effet secondaire d'un inhibiteur de kinase, dans lequel l'effet secondaire est au moins un effet choisi parmi le syndrome main-pied et un saignement.

13. Agent pour une utilisation en thérapie anticancéreuse selon la revendication 14, dans lequel l'inhibiteur de kinase est :
le 4-[4-[[4-chloro-3-(trifluorométhyl)phényl]carbamoyl-amino]phénoxy]-N-méthylpyridine-2-carboxamide,
le 4-[4-({[4-chloro-3-(trifluorométhyl)phényl]carbamoyl}-amino)-3-fluorophénoxy]-N-méthylpyridine-2-carboxamide,
la N-(2-chloro-4-((6,7-diméthoxy-4-quinazolinyl)oxy)-phényl)-N'-propylurée,
le N-[3-[5-(2-aminopyrimidin-4-yl)-2-tert-butyl-1,3-thiazol-4-yl]-2-fluorophényl]-2,6-difluorobenzenesulfonamide,
le chlorhydrate de 1-[1-[(2-aminopyridin-4-yl)méthyl]-indol-4-yl]-3-(5-bromo-2-méthoxyphényl)urée,
la N-[4-(3-amino-1H-indazol-4-yl)phényl]-N'-(2-fluoro-5-méthylphényl)urée,
la 1-[2-chloro-4-(6,7-diméthoxyquinoléin-4-yl)oxyphényl]-3-(5-méthyl-1,2-oxazol-3-yl)urée,
le 1-N'-[3-fluoro-4-[6-méthoxy-7-(3-morpholin-4-yl-propoxy)quinoléin-4-yl]oxyphényl]-1-N-(4-fluorophényl)-cyclopropane-1,1-dicarboxamide,
le N-[[3-fluoro-4-[2-(1-méthylimidazol-4-yl)thiéno[3,2-b]pyridin-7-yl]oxyphényl]carbamothioyl]-2-phénylacétamide,
le 1-N'-[2-fluoro-4-[2-[[4-(4-méthylpipérazin-1-yl)-pipéridine-1-carbonyl]amino]pyridin-4-yl]oxyphényl]-1-N-(4-fluorophényl)cyclopropane-1,1-dicarboxamide,
le 4-[3-chloro-4-[[(cyclopropylamino)carbonyl]amino]-phénoxy]-7-méthoxy-monométhanesulfonate,
le 1-N'-(4-fluorophényl)-1-N-[4-[[2-(2-morpholin-4-yl-éthylcarbamoyl)-1H-pyrrolo[2,3-b]pyridin-4-yl]oxy]phényl]-cyclopropane-1,1-dicarboxamide,
la 1-méthyl-5-[2-[5-(trifluorométhyl)-1H-imidazol-2-yl]-pyridin-4-yl]oxy-N-[4-(trifluorométhyl)phényl]-benzimidazol-2-amine,
le 6-[7-[(1-aminocyclopropyl)méthoxy]-6-méthoxyquinoléin-4-yl]oxy-N-méthylnaphtalène-1-carboxamide,
ou le 2-diméthyl-6-[7-(2-morpholin-4-yl-éthoxy)quinoléin-4-yl]oxy-1-benzofuran-3-carboxamide.
